# EUROPEAN PATENT APPLICATION

(11) **EP 1 949 853 A1**
(43) Date of publication of application: **30.07.2008**
(21) Application number: 06810769.7
(22) Date of filing: 28.09.2006
(51) Int. Cl.: A61B 5/022

(54) **BLOOD PRESSURE METER**

(30) Priority: 25.10.2005 JP 2005008855 U
(71) Applicant: TERUMO KABUSHIKI KAISHA, Tokyo 151-0072 (JP)
(72) Inventor: SASAGAWA, Hiroki, Fujinomiya-shi, Shizuoka 4180015 (JP); NARIMATSU, Kiyoyuki, Fujinomiya-shi, Shizuoka 4180015 (JP)
(74) Representative: Paget, Hugh Charles Edward
(86) International application number: PCT/JP2006/319322
(87) International publication number: WO 2007/049425

(57) **Abstract**

The sphygmomanometer comprises a cuff 2 for inserting the upper arm, pressurizing means 21 and 71 for pressurizing the cuff 2 by supplying air until blood pressure can be measured, detection means 24 and 72 for detecting information regarding blood vessels when the pressure within the cuff is controlled by the pressurizing means, determining means 71 for determining blood pressure, pulse rate and pulse pressure values from the information regarding blood vessels detected by the detecting means 24 and 72; displaying means 51 for displaying pulse rate and pulse pressure values; and control means 71 for controlling the display means 51 to display the pulse rate and pulse pressure values in line or alternately.

## Description

### TECHNICAL FIELD

The present invention relates to an arm insertion type sphygmomanometer of which a cuff is incorporated in a housing of the sphygmomanometer.

### BACKGROUND ART

Conventionally, a sphygmomanometer measures systolic (maximum), diastolic (minimum) blood pressure values, pulse rate, and pulse pressure, and displays measurement results on a display (for example, patent reference 1).
Patent reference 1: Japanese Patent Laid-Open No. H4-250132

### DISCLOSURE OF INVENTION

### PROBLEMS THE INVENTION IS TO SOLVE

According to the above mentioned prior arts, however, measurement results which are automatically displayed regardless of key operations and such are systolic pressure, diastolic pressure and pulse rate. In order to display pulse pressure, it is necessary to perform additional key operations or the like. For this reason, it would be convenient if pulse pressure were displayed automatically along with other measured values.

The present invention has as its objective to create a sphygmomanometer capable of displaying measurement results of both pulse rate and pulse pressure without requiring specific key operations.

### MEANS OF SOLVING THE PROBLEMS

In order to solve the above mentioned problems and to achieve the objective, the invention provides a sphygmomanometer characterized by comprising a cuff for inserting the upper arm; pressurizing means for pressurizing the cuff by supplying air until blood pressure can be measured; detection means for detecting information regarding blood vessels when the pressure within the cuff is controlled by the pressurizing means; determining means for determining blood pressure, pulse rate and pulse pressure values from the information regarding blood vessels detected by the detecting means; displaying means for displaying blood pressure, pulse rate and pulse pressure values; and control means for controlling the display means to display the pulse rate and pulse pressure values in line or alternately.

Further, the sphygmomanometer of the present invention is characterized by comprising a cuff for inserting the upper arm; pressurizing means for pressurizing the cuff by supplying air until blood pressure can be measured; detection means for detecting information regarding blood vessels when the pressure within the cuff is controlled by the pressurizing means; determining means for determining blood pressure from the information regarding blood vessels detected by the detecting means; displaying means for displaying blood pressure values; a storing means which stores the blood pressure values; and a prohibiting means which prohibits storage of the blood pressure values in the storing means.

Further, the sphygmomanometer of the present invention is characterized by comprising a cuff for inserting the upper arm; pressurizing means for pressurizing the cuff by supplying air until blood pressure can be measured; detection means for detecting information regarding blood vessels when the pressure within the cuff is controlled by the pressurizing means; determining means for determining blood pressure from the information regarding blood vessels detected by the detecting means; displaying means for displaying blood pressure values; and control means which executes a learning mode that adjusts the amount of pressure in the cuff at the time of measurement using measurement values of the past, where the control means determines the cuff pressure from the most recently measured systolic pressure or the average of more than 3 times systolic pressures measured in the past.

Further, preferably, the displaying means displays the pulse rate value and the pulse pressure value in line on a different area of the display.

Further, preferably, the displaying means displays the pulse rate value and the pulse pressure value alternately on same area of the display.

Further, preferably, a main body unit having the display is detachable to a cuff unit having a housing provided with the cuff and a pump supplying air into the cuff.

Further, preferably, the information regarding blood vessels is information relating to either Korotkoff sounds and vibrational pulse waves.

### EFFECTS OF THE INVENTION

As mentioned above, according to the present invention, it is possible to display measurement results of both pulse rate and pulse wave without having to make specific key operations, thus improving convenience.

Further features of the present invention will become apparent from the following description of exemplary embodiments with reference to the attached drawings.

### BRIEF DESCRIPTION OF DRAWINGS

The accompanying drawings, which are incorporated in and constitute a part of the specification, illustrate embodiments of the invention and, together with the description, serve to explain the principles of the invention.

Fig. 1A is a perspective exterior view, seen from the front, of the sphygmomanometer according to an embodiment of the present invention.
Fig. 1B is a perspective exterior view, seen from the side, of the sphygmomanometer according to an embodiment of the present invention.
Fig. 2A is a perspective exterior view showing a stored position of an armrest of the cuff unit according to the present embodiment.
Fig. 2B is a perspective exterior view showing an open position of the armrest of the cuff unit according to the present embodiment.
Fig. 3A is a perspective view illustrating attachment/detachment of a main body unit to a cuff unit according to the present embodiment.
Fig. 3B is another perspective view illustrating attachment/detachment of the main body unit from the cuff unit according to the present embodiment.
Fig. 4A is a frontal view of the main body unit according to the present embodiment.
Fig. 4B is a top view of the main body unit according to the present embodiment.
Fig. 4C is a rear view of the main body unit according to the present embodiment.
Fig. 5A is a block diagram of a cuff unit in the sphygmomanometer according to the present embodiment.
Fig. 5B is a block diagram of a main body unit in the sphygmomanometer according to the present embodiment.
Fig. 6 is a flowchart showing the blood pressure measuring operation by the sphygmomanometer according to the present embodiment.
Fig. 7 is a flowchart showing the blood pressure measuring operation by the sphygmomanometer according to the present embodiment.
Fig. 8 is a flowchart showing the procedure of battery check by the sphygmomanometer of the present embodiment.
Fig. 9A is a display example of measurement results on the main body unit according to the present invention.
Fig. 9B is a display example of measurement results on the main body unit according to the present invention.

### DESCRIPTION OF NUMERALS

1 sphygmomanometer
2 cuff
3 housing
4 opening
5 adaptor socket
6 arm rest
7 hinge
8 protrusion
9 groove
10 cuff unit
11 start switch (SW)
12 stop (emergency discharge) switch (SW)
13 memory prohibition switch (SW)
14 AC adapter inserting slot
15 battery storage
21 pump
22 depressurizing control unit
23 force discharge unit
24 microphone
25 noise sensing unit
26 cuff unit power supply
27 AC adapter
50 main body unit
51 first display
52 second display
53 averaging switch (SW)
54 graph shift switches (SW)
55 display ON switch (SW)
56 user change switch (SW)
57 monthly display switch (SW)
58 display change switch (SW)
59 battery storage
60 reset switch (SW)
61 time/alarm setting change switch (SW)
62 alarm on/off switch (SW)
71 control unit
72 pressure detecting unit
73 filter amp unit
74 pump driving unit
75 depressurization valve driving unit
76 discharge valve driving unit
77 filter amp unit
78 main body unit power supply
79 power supply control unit
80 clock circuit
82 buzzer
83 communication unit

### BEST MODE FOR CARRYING OUT THE INVENTION

Embodiments of the present invention will be explained in detail using figures included herein.
It is to be understood that the embodiments explained below are merely examples for realizing the present invention, and thus the present invention is applicable to varied arrangements of the embodiments which are within the original intention.

### Explanation of the device

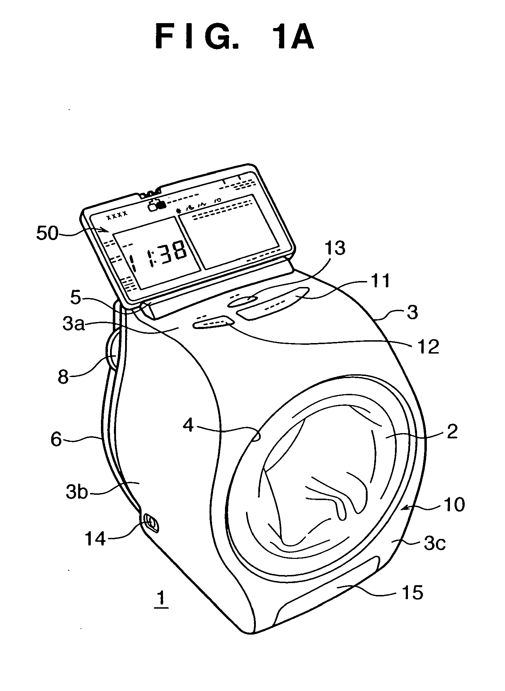

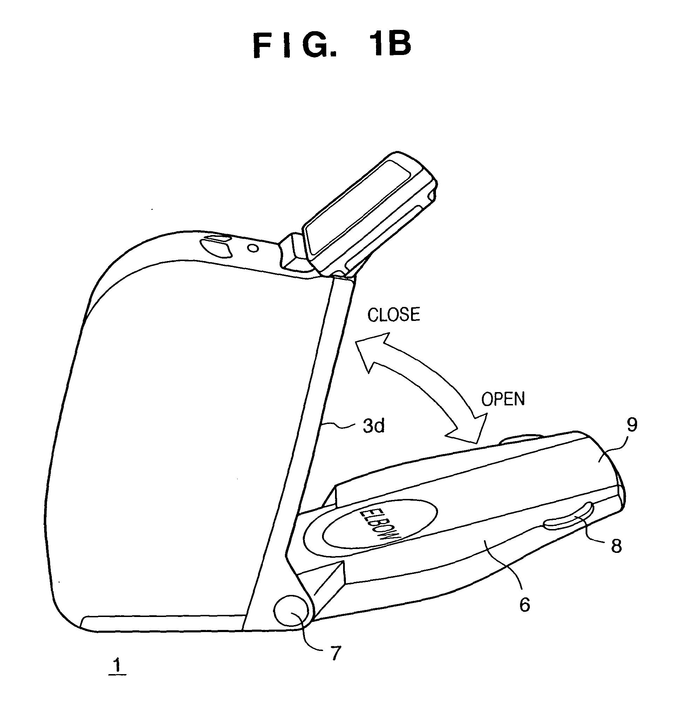

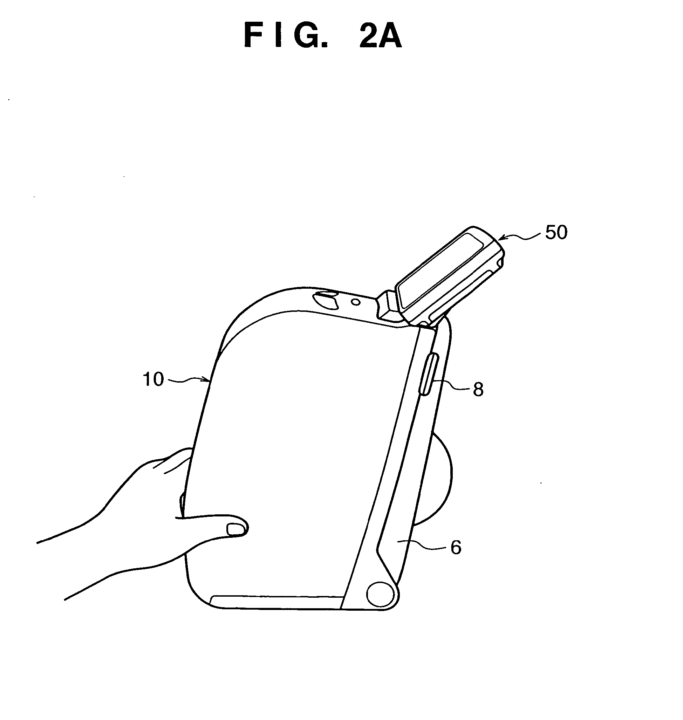

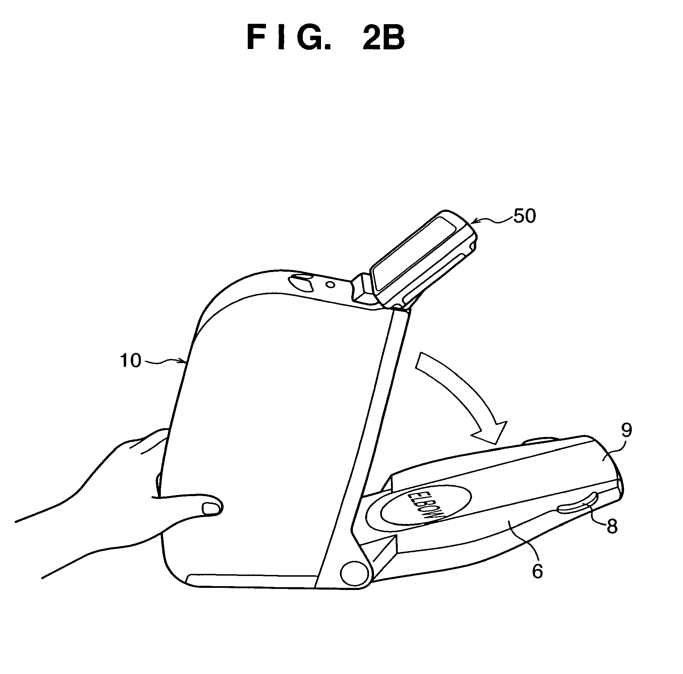

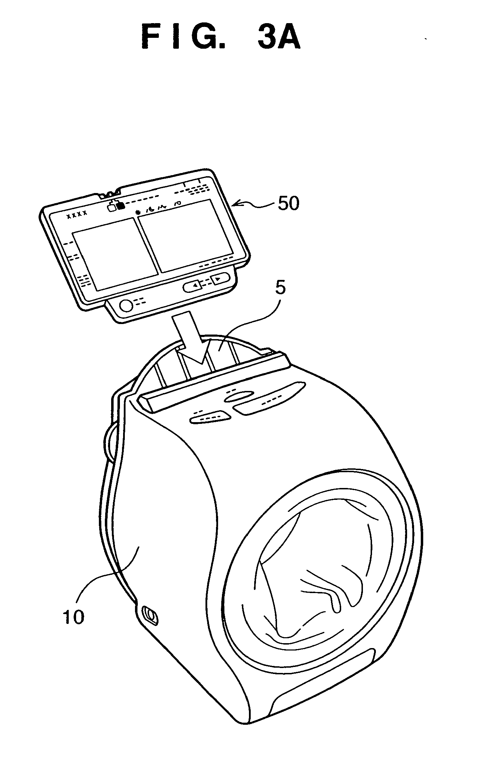

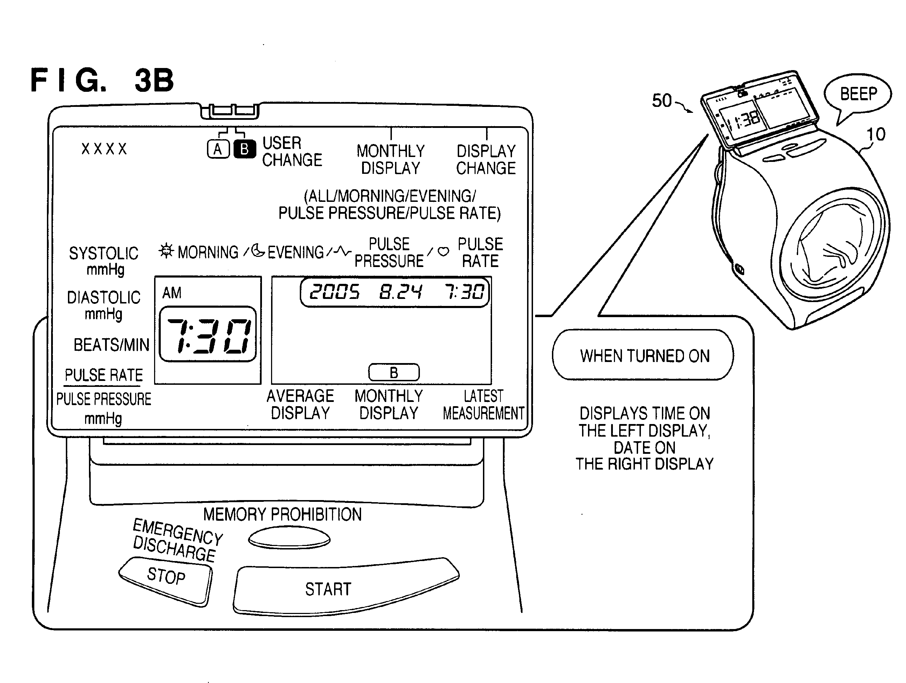

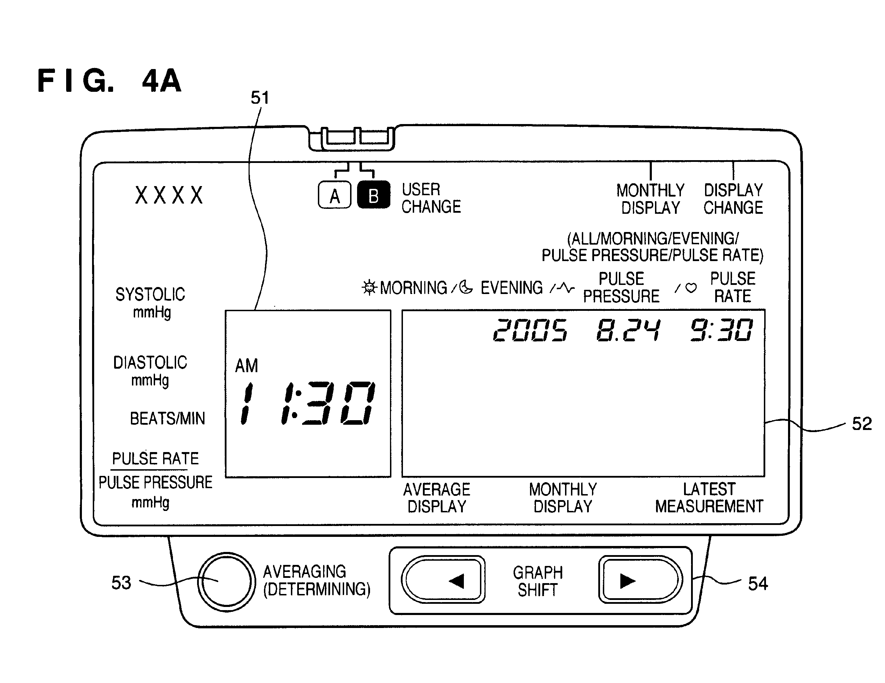

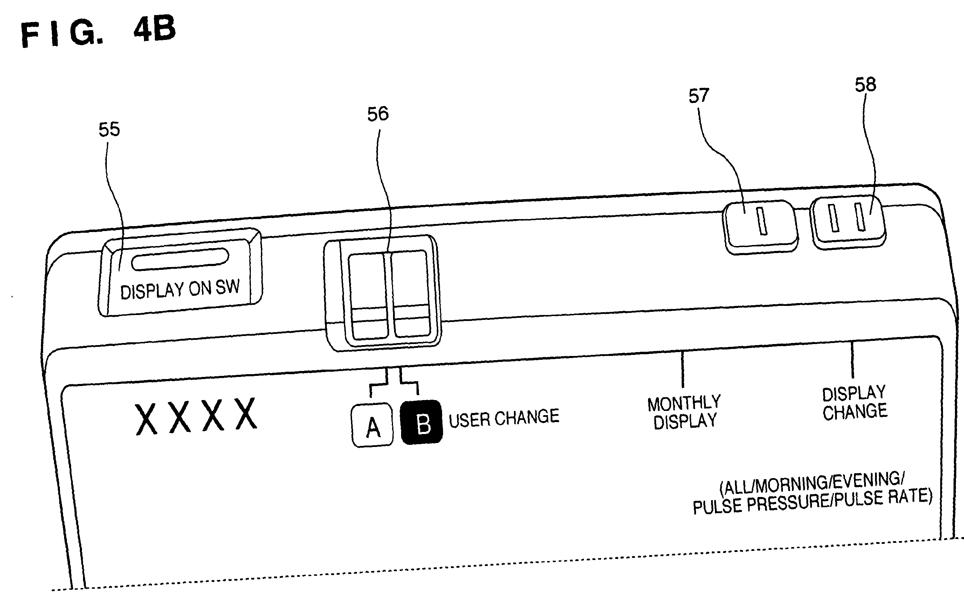

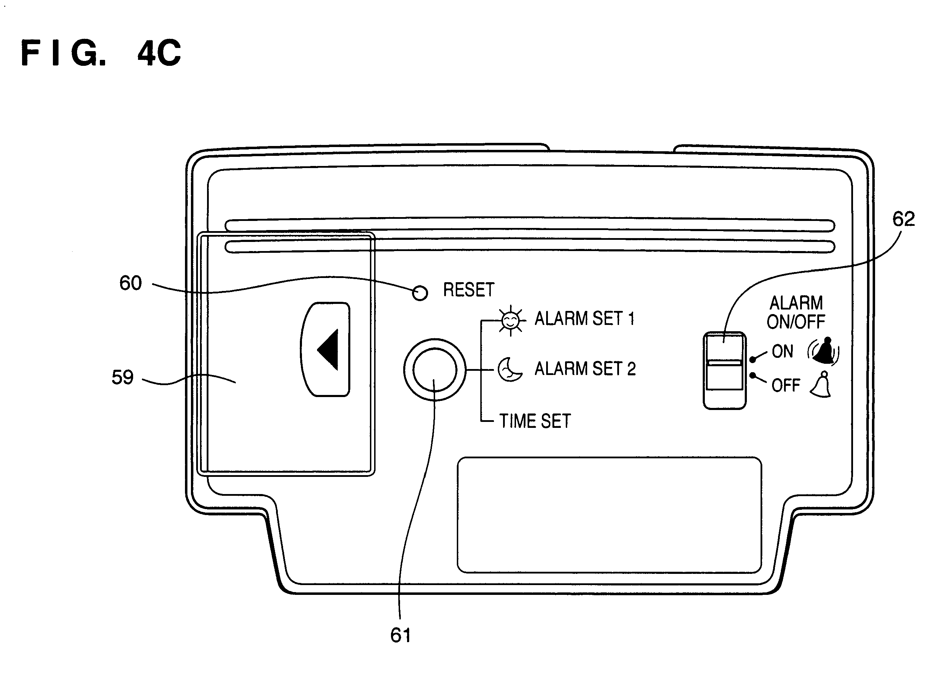

Fig. 1A is a perspective exterior view, seen from the front, of the sphygmomanometer according to an embodiment of the present invention. Fig. 1B is a perspective exterior view, seen from the side, of the sphygmomanometer according to an embodiment of the present invention. Fig. 2A is a perspective exterior view showing a stored position of an armrest of the cuff unit according to the present embodiment. Fig. 2B is a perspective exterior view showing an open position of the armrest of the cuff unit according to the present embodiment. Fig. 3A is a perspective view showing attachment/detachment of a main body unit to a cuff unit according to the present embodiment. Fig. 3B is another perspective view showing attachment/detachment of the main body unit from the cuff unit according to the present embodiment. Fig. 4A is a frontal view of the main body unit according to the present embodiment. Fig. 4B is a top view of the main body unit according to the present embodiment. Fig. 4C is a rear view of the main body unit according to the present embodiment.

As illustrated in Figs. 1A to 4C, in a sphygmomanometer 1 of the current embodiment, a cuff 2 which pressurizes (compresses) the brachium during blood pressure measurements comprises a cuff unit 10 built into a housing 3 having a predetermined outer shape (longitudinal) which can stand on its own, and a main body unit 50 which can be attached to and detached from the cuff unit 10. Unlike conventional devices, the present invention is an arm insertion-type sphygmomanometer which does not require wrapping the band around the brachium of the examinee, and can measure blood pressure with either the left or the right arm.

The housing 3 has an opening 4 into which the examinee inserts his arm, and the inner surface of the opening 4 is laid with the cuff 2. The cuff 2 is made from a deformable material such as fabric or rubber sacs that forms an insertion hole for the brachium. Arranged on a top surface 3a of the housing 3 are an adaptor socket 5 for attachment and detachment of the main body unit 50 as well as various operational units such as a start switch (SW) 11, a stop (emergency discharge) switch (SW) 12 and a memory prohibition switch (SW) 13. Further, an AC adaptor inserting slot 14 is built on a side surface 3b of the housing 3, and a battery storage 15 is built on a front surface 3c.

Further, an armrest 6 on which the examinee's elbow rests, is built on a rear surface 3d of the housing. This armrest 6 is rotatably attached onto hinges 7 located on the rear bottom portion of the housing 3 such that it could be closed to a stored position as shown in Fig. 2A where the cuff 2 is closed, and opened to an opened position as shown in Fig. 2B to accommodate the examinee's arm.

Further, a groove 9 running from the hinges 7 to the free (distal) end is formed on the support surface (top surface) of the armrest 6 which accommodates the examinee's arm, and serves a function of correctly positioning the brachium.

Further, the rear surface 3d of the housing 3 is shaped to fit the exterior shape of the armrest 6, which is flat and smooth and therefore integrate with the outer shape of the housing 3 without any protrusions when closed into the stored position as shown in fig. 2B.

The armrest 6 is opened and closed with a click stop by forming cam-like structures on the hinges 7 (locking into the armrest 6), thereby prohibiting the armrest from falling down from the stored position to an opened position when not in use and maintaining the stored position. The armrest 6 can be opened to the opened position by pushing backward protrusions 8 formed on the armrest 6 while holding down the cuff unit 10. With these features, it is possible to store the device in a compact configuration and save space when not in use.

The cuff unit 10 and the main body unit 50 are provided with connectors 9 and 49, respectively. When the main body unit 50 is inserted into the adaptor socket 5 of the housing 3, it is connected electronically to the cuff unit 10 and also physically locked into a configuration that prohibits leakage of air from the cuff 2 and a pressure detecting unit 72.

Further, connectors 9 and 49 both have electric power supply ports supplying electric power from the cuff unit 10 to the main body unit 50, signal ports conveying control signals between the two units, and connection verifying ports which verify connection of the cuff unit 10 and the main body unit 50. When the main body unit 50 is properly inserted into the adapter socket 5 of the housing 3, electronic connection with the cuff unit 10 is established, leading to supply of power, display of time and date and generation of a beep sound. The adapter socket 5 holds the main body unit 50 at an angle tilted rearward to the top surface 3a of the housing 3.

Arranged side by side on the front surface of the main body unit 50 are a first display 51 which displays systolic and diastolic blood pressure, pulse rate and pulse pressure, and a second display 52 which simultaneously displays a graph of the past results (blood pressure value and measurement date) of predetermined times of measurements, such as the most recent 20 times measurements. Both the first display and the second display are arranged parallel and made from LCD panels. Further, the lower front portion of the main body unit 50 is arranged with an averaging switch (SW) 53 for displaying the average blood pressure value of the most recent measurements, such as the most recent 20 times measurements, graph shift switches (SW) 54 which allow selecting any arbitrary graph out of a plurality of bar graphs displaying the most recent systolic and diastolic blood pressure values successively. The averaging switch (SW) 53 doubles as determining switch for setting a time or the like.

Further, the top surface of the main body unit 50 is arranged with a display ON switch (SW) 55 which controls ON/OFF of the power supply as well as those of the display 51 and the display 52, a user change switch (SW) 56 which changes examinees, a monthly display switch (SW) 57 which displays monthly averages of blood pressure values, and a display change switch (SW) 58 which changes each display or all displays of blood pressure values, pulse rates and pulse rates measured in the morning and in the evening. The user change switch 56 can change between A and B (therefore 2 users), and displays measurement results and graphs of the selected user.

Further, on the rear surface of the main body unit 50, there are a battery storage 59, a reset switch (SW) 60 which resets the main body unit 50 and deletes stored measurement data, a time/alarm setting change switch (SW) 61 for setting the clock or the alarm, and an alarm ON/OFF switch (SW) 62 which rings the alarm at a preset time informing the examinee of a measurement time.

Each of these said functions of the main body unit 50 is attainable when the main body unit 50 is attached to or even detached from the cuff unit 10.

The first and second displays 51 and 52 not only display measurement values and trend graphs, but also flash-display graphs being measured and characters indicating that the measurement is in progress. Further, other display items indicating pressurizing and depressurizing (discharge) etc., are also displayed.

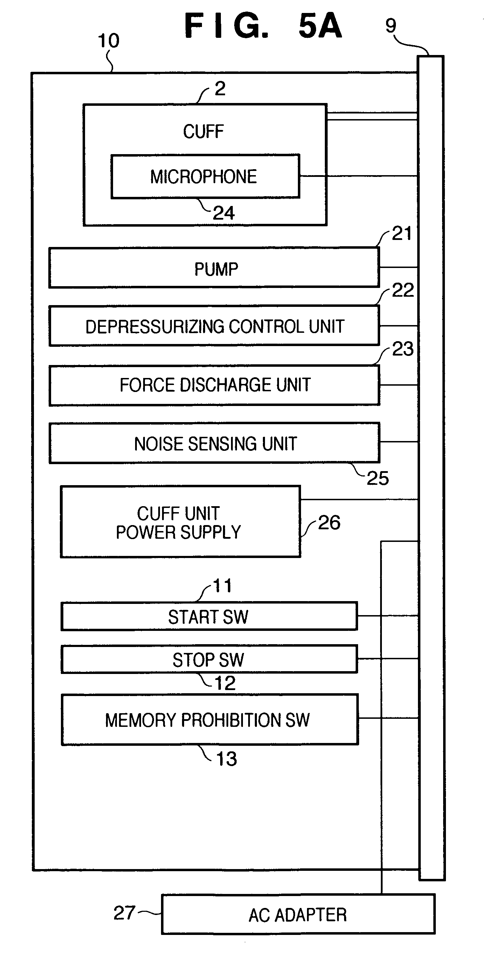

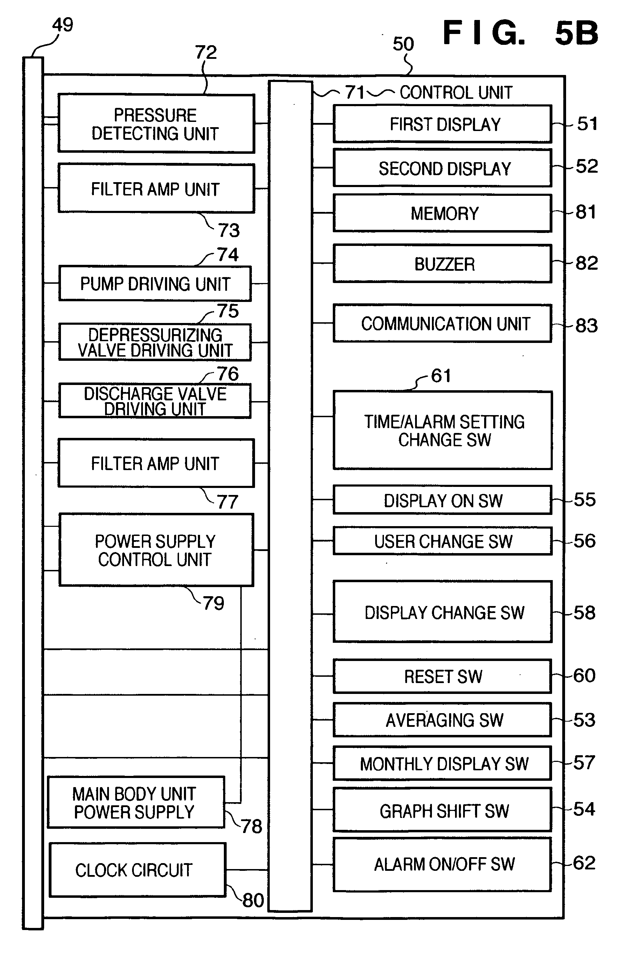

Figs. 5A and 5B are block diagrams showing the sphygmomanometer according to an embodiment of the present invention.

As shown in Figs. 5A and 5B, the cuff unit 10 comprises a pump 21 which supplies air to the cuff 2, a depressurizing control unit 22 which depressurizes (discharges) air in the cuff 2 at a constant speed, a force discharge unit 23 which forcibly discharges air from the cuff 2, a microphone(s) 24 for detecting sounds generated by the blood flow of the examinee, and a noise canceling unit 25 which detects vibrational noise coming from the outside and entering the microphone(s) 24. The microphone(s) 24 is built into the cuff 2. The noise sensing unit 25 is comprised of a vibration sensor such as a buzzer plate, and detects various types of vibration, such as those transmitted from the measurement platform installed in the cuff unit 10 or generated when physical contacts are made.

Further, when measuring blood pressure using Korotkoff sounds, a pair of the microphones 24 are positioned facing each other on the opening 4 in the cuff 2. One of the microphone 24 is positioned near the arteria of the brachium and the other microphone 24 is positioned far from the arteria of the brachium. Further, a vibration sensor is placed on a portion of the housing 3 which is closer to one of the microphones 24 (which is far from the arteries of the brachium), and it is thus possible for one of the microphones 24 to handle vibrational noise much like the vibration sensor. For this reason, it is possible to utilize this particular microphone 24 as a dual sensor detecting vibration, and remove the vibration sensor.

Further, in addition to the start switch 11, the stop switch 12 and the memory prohibition switch 13, the cuff unit 10 also equipped with the cuff unit power supply 26 and the AC adapter 27. The cuff unit power supply 26 is comprised of a plurality (4 pieces) of AAA dry-batteries stored in said battery storage 15, and comprises a direct current power supply of 6V DC. The AC adapter 27 is connected to said AC adapter inserting socket 14 and comprises an alternating current of 100V rating.

On the other hand, a control unit 71 is mounted in the main body unit 50. Also, the control unit 71 is connected with a pressure detecting unit 72 which detects pressure within the cuff 2, a filter amp unit 73 which signal-processes signals detected by the microphones 24, a pump driving unit 74 which drives the pump 21, a depressurization valve driving unit 75 which drives the depressurizing valve of the depressurizing control unit 22, a discharge valve driving unit 76 which drives the discharge valve of the force discharge unit 23, a filter amp unit 77 which signal processes detected signals from the noise sensing unit 25, a main body unit power supply 78, the cuff unit power supply 26, the AC adapter 27, a power supply control unit 79 which controls the main body unit power supply 78, a clock circuit 80 which has a clock function, a memory 81 comprised of a non-volatile memory or the like which stores blood pressure values and measurement time data, a buzzer 82 which generates alarm sounds, and a communication unit 83 which controls, with a RS232C or the like, communication with external devices such as personal computers and the like.

The pressure detecting unit 72 converts and amplifies the interior pressure of the cuff 2 into an electronic signal, and further converts into a digital signal by A/D conversion which is then outputted to the control unit 71. The digital signal inputted to the control unit 71 is converted to pressure values in the order of precedence and displayed on the displays 51 and 52 as display signals.

After the conversion and amplification of signals detected by the microphones 24 to electric signals, the filter amp unit 73 further converts the signals to digital signals by A/D conversion and outputs to the control unit 71. When measuring the blood pressure using Korotkoff sounds, the control unit 71 calculates blood pressure values (systolic and diastolic pressure) by detecting Korotkoff (K) sounds and its generation (start) and disappearance (end) points from the inputted digital signals, and outputs to the displays 51 and 52 as display signals. When using pressure pulse waves, systolic and diastolic pressure values are calculated with known pressure detection and pulse detection & calculation, and displayed on the displays 51 and 52.

Further, the control unit 71 calculates pulse rate from the blood vessel pulses (vibrational pulse wave) and/or the intervals in the K sounds, and displays along with display signals on the display 51 and 52.

Further, the control unit 71 calculates pulse pressure by subtracting the diastolic pressure from the systolic pressure, and outputs display signals to the display 51 to be displayed simultaneously in adjacent fields or alternately in a same field.

The filter amp unit 77 filters out the noise components of the signals detected at the noise sensing unit 25 and also amplifies the filtered signal components which then are sent to the control unit 71.

The pump driving unit 74, the depressurizing valve driving unit 75 and the discharge valve driving unit 76 each drive the pump 21, the depressurizing control unit 22 and the force discharge unit 23, respectively, based on the control signals from the control unit 71.

The power supply control unit 79 switches power supply type components in order to utilize the AC adapter 27 or the cuff unit power supply 26 (which is preferably used) when the main body unit 50 is connected to the cuff unit 10, and to utilize the main body unit power supply 78 only when the connection to the cuff unit 10 to the main body unit is not made.

The main body unit power supply 78 comprises a plurality (2 pieces) of lithium button batteries stored in said battery storage 59, and is used mostly as a backup power supply for the memory 81.

The memory 81 stores each of the measurement values of users A and B, set using the user change switch 56, in different fields of the display. Further, the memory 81 reads out and displays each of the measurement values of users A and B, set using the user change switch 56. The memory 81 has a capacity which can store data of approximately 730 measurements (when measured twice daily in the morning and the evening, 3 years of raw data, or 60 months of monthly average data can be stored).

Further, the control 71 is connected to the first display 51, the second display 52, the averaging switch 53, the graph shift switches 54, the display ON switch 55, the user change switch 56, the display change switch 58, the reset switch 60, the time/alarm setting change switch 61, and the alarm ON/OFF switch 62.

The control unit 71 is installed with a CPU, a ROM, a RAM, an interface circuit and so on. The present embodiment utilizes the Riva-Rocci Korotkoff method as the method of measuring blood pressure, but other methods of measurements such as pulse wave measurements (oscillometric method) can also be utilized, in which case the microphones 24 are not required. The control unit 71 inputs operation signals from each of said switches in accordance with the blood pressure measurement program stored in the ROM. Subsequently, the control unit 71 outputs driving signals to the pump driving unit 74, the depressurizing valve driving unit 75, and the discharge valve driving unit 76 based on detected signals from sensors 24, 25 and 72. Further, the control unit 71 acquires blood pressure values and pulse rate from the K sound signals of the microphones 24, and stores these values in the memory 81 and outputs display signals to the displays 51 and 52. Further, the control unit 71 cancels the K sound signals detected from the microphones 24 and deletes the effect of vibrational noise when the detected vibrational noise by the noise sensing unit 25 is above a predetermined level.

The main body unit 50 can be connected, via the communication unit 83, to each of the measuring instruments including external devices such as a personal computer and a printer, a thermometer, a weighing scale, a blood sugar measuring device, a pedometer, etc. It is possible to store each of the measurement data from these measuring instruments in the memory 81, output to a printer, or to download to a personal computer. Therefore, for example, a physician can easily print out or download to his computer the data such as blood pressure values measured by the examinee at his own residence.

### (explanation of the operation)

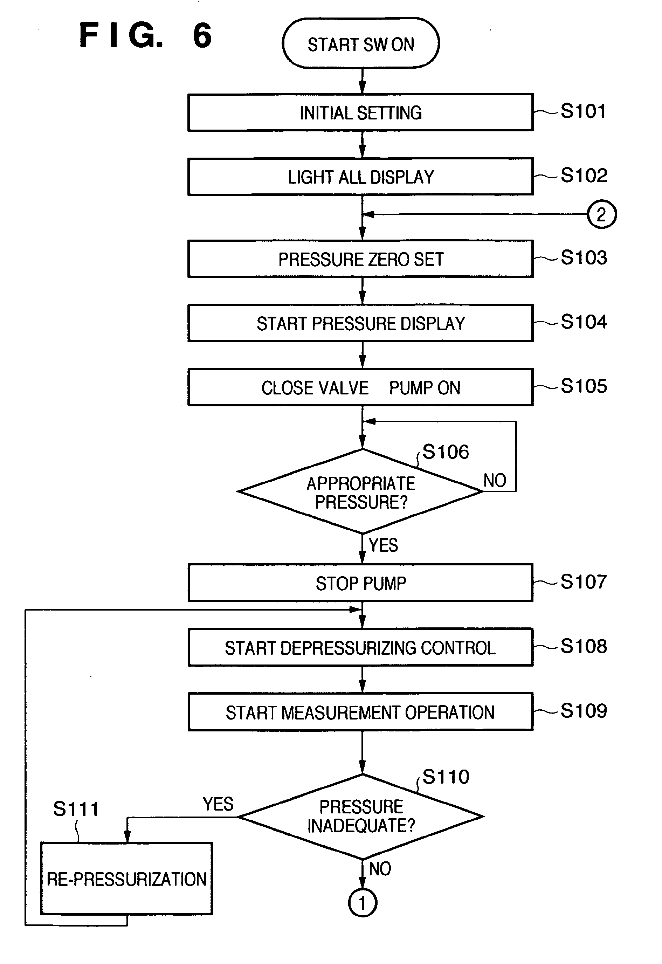

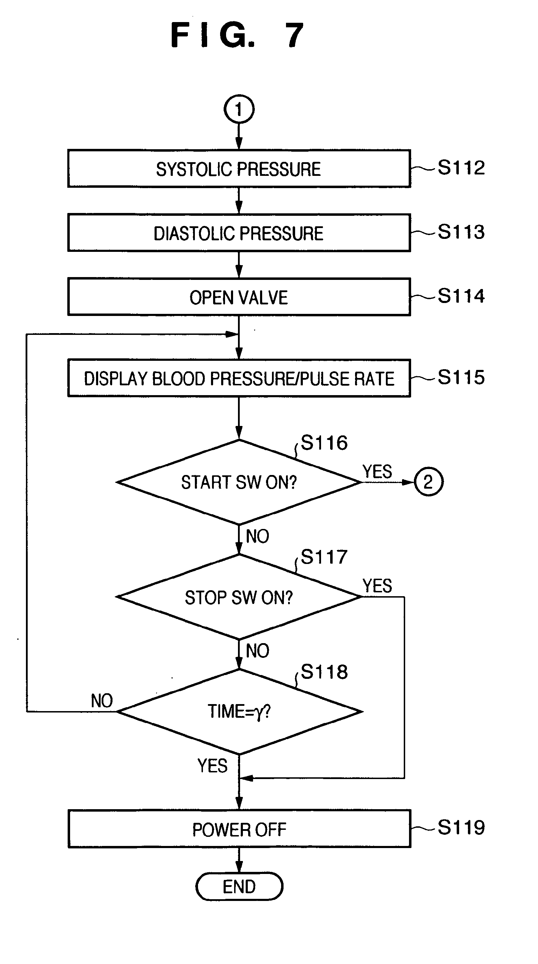

Figs. 6 and 7 are flow charts showing the operation of blood measurement in the sphygmomanometer according to an embodiment of the present invention.

The steps listed below are attained by the CPU of the control unit 71 executing a program stored in the ROM.

In Fig. 6, when the start switch 11 is pressed, initial setting is carried out (S101). Next, the displays 51 and 52 are checked (all segments are lightened for about one second) (S102). Next, it is determined that inner pressure is not added to the cuff 2 and pressure value is set to be zero in this state (S103). Pressure display is started on the display 51 and 52 (S104).

Next, the discharge valve of the force discharge unit 23 is closed and the pressurization is started by operating the pump 21 (S105). Next, the pump 21 pressurizes the cuff 2 until the pressure within the cuff 2 reaches an appropriate pressure value (S106), at which point pressurization by the pump 21 stops (S107). Next, depressurizing control unit 22 initiates depressurization (S108), and the operation of blood pressure measurement starts (S109). Further, if pressure is determined to be inadequate during depressurization control, re-pressurization takes place (S111), and depressurization is initiated once again (S108).

In S112 of Fig. 7, the K sound signals are detected, and the pressure at which the first K sound signal is detected is set as the systolic pressure. Further, depressurization is carried on until the K sound signals are no longer detected, and the pressure when the very last K sound signal is detected is set as the diastolic pressure. Further, in S113, the pulse rate value is calculated from the blood vessel pulsation and/or the intervals between the K sounds which can be obtained from said blood pressure values.

When the diastolic blood pressure is set, the discharge valve of the force discharge unit 23 is opened and the depressurization control is stopped (S114), and the blood pressure value and the pulse rate are simultaneously displayed (S115). If further measurements are to be made, the start switch 11 is pressed (S116), and either displays the measurement results in S118 if the stop switch 12 is not pressed, or jumps to S119 and turns the power off if the stop switch 12 is pressed.

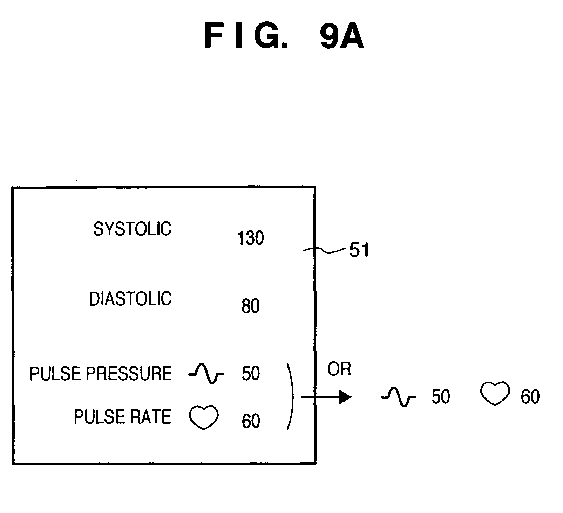

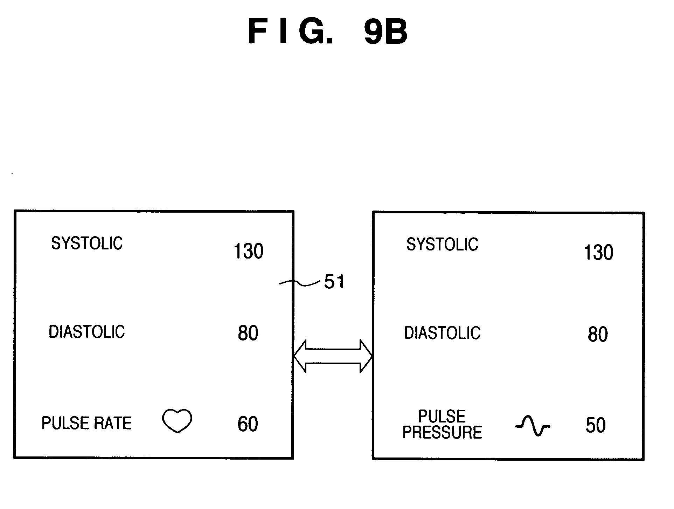

At said S115, as shown in Fig. 9A, the first display 51 displays systolic pressure, diastolic pressure and pulse rate measured from the present round of measurement, and at the same time displays the pulse pressure (pulse mark as a character display) along with the pulse rate longitudinally or transversally in line on a different area of the display. As shown in Fig. 9B, the pulse pressure and pulse rate may be displayed on the same area of the first display 51 in a manner that the pulse pressure and pulse rate are displayed and switched alternately on the same area at a predetermined time (second) intervals.

Also, after a lapse of a certain amount of time (seconds) γ without the stop switch 12 being pressed, the power is switched off and the present process flow is terminated (S117-5119).

### (Memory prohibition function)

After displaying measurement results in above S115, if the memory prohibition switch 13 is pressed and the time since display of measurement values has not passed a predetermined time length β (seconds), processing to prohibit storage of measurement values in the memory 81 proceeds. Visually, if the memory prohibition switch 13 is pressed while the graph showing the current measurement results is flashing on the second display 52 (for 3 minutes, for example), display of the graph from the current measurement will be stopped and disappear from the screen. By pressing the memory prohibition switch 13 after pressing the start switch 11 (after start of measurement) or within a certain amount of time since the initial display of the measurement result, blood pressure measurements of individuals other than the main user can be prohibited from being stored, allowing storage and accumulation of measurement results of only the main user, and also allows other individuals to use the sphygmomanometer.

### (Pressurization learning mode)

Further, the control unit 71 executes a pressurization learning mode.

The control unit 71 reads out the systolic pressure previously measured for each user set by the user change switch 56. Consequently, if the pressure value A during systolic pressure measurement is lower than a predetermined value A1, the control unit 71 supplies additional pressure of an adjustment value α, and stops pressurizing when the pressure reaches a new value of A+α by controlling the pump 21 via the pump driving unit 74. Also, instead of the above pressure adjustment process, if the start switch 11 was pressed (or if the alarm was set by the switch 61 to ring) in the morning (for example 5 to 9AM), a pressure value B which is the average of several systolic pressure values previously measured in the morning (for example, the average value of the last 3 to 10 measurements) can be adjusted by adding an additional value of β. Likewise, if the measurements were made in the evening (for example 5 to 9PM), a pressure value B which is the average of several systolic pressure values previously measured in the evening (for example, the average value of the last 3 to 10 measurements) can be adjusted by adding an additional value of β. Subsequently, when the pressure reaches a new value of B+β, the control unit 71 can stop pressurizing by controlling the pump 21 via the pump driving unit 74.

### (Battery check function)

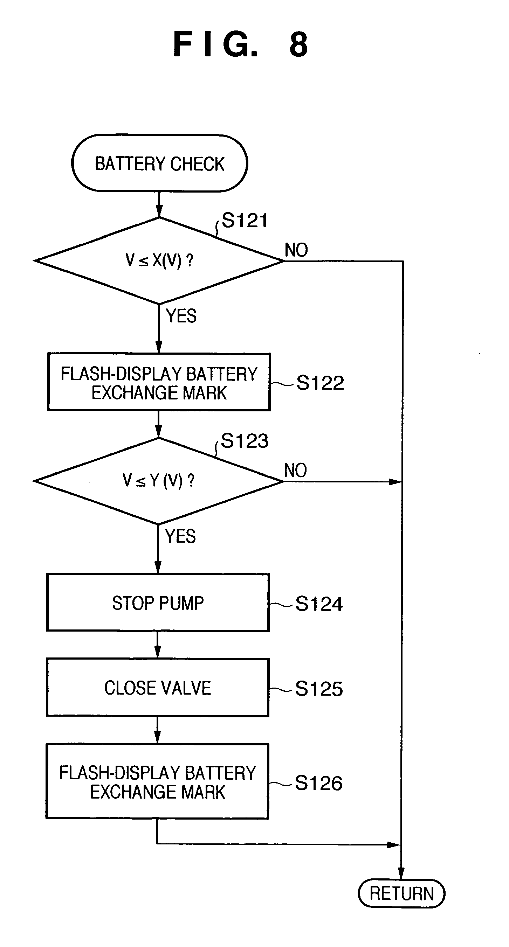

The control unit 71 executes battery check illustrated in figure 8 during the initial setting of S101.

It is shown in figure 8 that when the output voltage V is lower than a predetermined value X (3.7± 0.2V), a battery exchange mark (S121, S122) is flash-displayed. Further, when the output voltage V is lower than another predetermined value Y (X-0.1±0.05V), the pump 21 is stopped, air from the cuff 2 is quickly discharged by opening the discharge valve of the force discharge unit 23, and a battery exchange mark (S123 to S126) is flash-displayed.

According to the above embodiments, even without special display operations, it is possible to display measurement results of both pulse rate and pulse pressure, resulting in improved convenience.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. A sphygmomanometer **characterized by** comprising:
a cuff for inserting the upper arm;
pressurizing means for pressurizing said cuff by supplying air until blood pressure can be measured;
detection means for detecting information regarding blood vessels when the pressure within said cuff is controlled by said pressurizing means;
determining means for determining blood pressure, pulse rate and pulse pressure values from the information regarding blood vessels detected by said detecting means;
displaying means for displaying blood pressure, pulse rate and pulse pressure values; and
control means for controlling said display means to display said pulse rate and pulse pressure values in line or alternately.

2. The sphygmomanometer according to claim 1 **characterized in that**, said displaying means displays said pulse rate value and said pulse pressure value in line on a different area of the display.

3. The sphygmomanometer according to claim 1 **characterized in that**, said displaying means displays said pulse rate value and said pulse pressure value alternately on same area of the display.

4. The sphygmomanometer according to claim 1 **characterized in that**, a main body unit having said display is detachable to a cuff unit having a housing provided with said cuff and a pump supplying air into said cuff.

5. The sphygmomanometer according to anyone of claims 1 to 4 **characterized in that**, said information regarding blood vessels is information relating to either Korotkoff sounds and vibrational pulse waves.

6. A sphygmomanometer **characterized by** comprising:
a cuff for inserting the upper arm;
pressurizing means for pressurizing said cuff by supplying air until blood pressure can be measured;
detection means for detecting information regarding blood vessels when the pressure within said cuff is controlled by said pressurizing means;
determining means for determining blood pressure from the information regarding blood vessels detected by said detecting means;
displaying means for displaying pulse rate and pulse pressure values determined by said determining means;
a storing means which stores the blood pressure values; and
a prohibiting means which prohibits storage of the blood pressure values in the storing means.

7. A sphygmomanometer **characterized by** comprising:
acuff into for inserting the upper arm;
pressurizing means for pressurizing said cuff by supplying air until blood pressure can be measured;
detection means for detecting information regarding blood vessels when the pressure within the cuff is controlled by said pressurizing means;
determining means for determining blood pressure from the information regarding blood vessels detected by said detecting means;
displaying means for displaying blood pressure values determined by said determining means;
and control means which executes a learning mode that adjusts the amount of pressure in the cuff at the time of measurement using measurement values of the past,
where said control means determines the cuff pressure from the most recently measured systolic pressure or the average of more than 3 times systolic pressures measured in the past.
